# EUROPÄISCHE PATENTSCHRIFT

(11) **EP 0 719 116 B1**
(45) Veröffentlichungstag und Bekanntmachung des Hinweises auf die Patenterteilung: **28.06.2000**
(21) Anmeldenummer: 95911277.2
(22) Anmeldetag: 01.03.1995
(51) Int. Cl.: A61B 17/70

(54) **VERANKERUNGSELEMENT UND JUSTIERWERKZEUG FÜR EIN SOLCHES**
ANCHORAGE COMPONENT AND ADJUSTING DEVICE THEREFOR
ELEMENT D'ANCRAGE ET SON DISPOSITIF DE REGLAGE

(30) Priorität: 18.07.1994 DE 4425357
(43) Veröffentlichungstag der Anmeldung: 03.07.1996
(73) Patentinhaber: Biedermann, Lutz, 78048 Villingen-Schwenningen (DE); Harms, Jürgen, Prof. Dr., 76337 Waldbronn (DE)
(72) Erfinder: Biedermann, Lutz, 78048 Villingen-Schwenningen (DE); Harms, Jürgen, Prof. Dr., 76337 Waldbronn (DE)
(74) Vertreter: Prüfer, Lutz H., Dipl.-Phys.
(86) Internationale Anmeldenummer: EP9500742
(87) Internationale Veröffentlichungsnummer: WO9602198

(56) Entgegenhaltungen:
- EP-A- 0 348 272
- EP-A- 0 441 729
- EP-A- 0 487 895
- DE-A- 4 234 118
- DE-C- 4 307 576

## Beschreibung

Die Erfindung betrifft ein Verankerungelement nach dem Oberbegriff des Patentanspruches 1 und ein Justierwerkzeug für ein solches.

Ein derartiges Verankerungselement ist aus der DE 42 34 118 A bekannt. Dieses Verankerungselement ist hervorragend zur Verbindung mit einem zu tragenden Stab geeignet. Zum Nachjustieren muß bei diesem Verankerungselement das Mutternelement und das Fixierelement vorübergehend gelöst werden, damit eine Relativverschiebung zwischen Verankerungselement und Stab möglich wird. Aus der DE 37 22 590 C ist eine Positionierungsvorrichtung zum Stabilisieren von Wirbelsäulensegmenten bekannt, bei der der Stab als Gewindestange ausgebildet ist und die Fixierung zwischen Stab und Verankerungselement durch zwei außen an dem Verankerungselement angreifende Muttern, die auf der Gewindestange verstellbar sind, erfolgt. Zum Zwecke einer Nachjustierung der Position zwischen Stab und Verankerungselement werden die Muttern entsprechend auf der Gewindestange verdreht.

Aufgabe der Erfindung ist es, ein Verankerungselement der eingangs beschriebenen Art und ein Justierwerkzeug für ein solches zu schaffen, mit dem sich einerseits eine gute Verbindung zwischen Verankerungselement und Stab erzielen läßt und andererseits sich eine leicht handhabbare Justiermöglichkeit ergibt.

Aus der DE-C-4 307 576 ist ein Verankerungselement nach dem Oberbegriff des Patentanspruches 1 bekannt.

Aus der EP-A-0 441 729 ist ein Verankerungselement mit einem zur Knochenverankerung bestimmten Schaft und mit einem Stab verbindbaren Kopf mit einem im wesentlichen U-förmigen Querschnitt, der an seinem Grund mit dem Schaft verbunden ist und zwei einen Kanal zur Aufnahme des Stabes bildende freie Schenkel hat, wobei die Schenkel ein Innengewinde aufweisen und wobei ein mit dem Innengewinde der Schenkel zusammenwirkendes Gewinde aufweisendes Fixierelement vorgesehen ist und wobei ein auf den aufzunehmenden Stab einwirkendes Fixierelement vorgesehen ist, bekannt.

Diese Aufgabe wird durch das in Patentanspruch 1 gekennzeichnete verankerungselement bzw. durch das in den Patentansprüchen 28 bzw. 39 gekennzeichnete Justierwerkzeug gelöst.

Weiterbildungen der Erfindung sind in den Unteransprüchen gekennzeichnet.

Weitere Merkmale und Zweckmäßigkeiten der Erfindung ergeben sich aus der Beschreibung eines Ausführungsbeispieles anhand der Figuren.

Von den Figuren zeigen:
- Fig. 1: eine Darstellung eines Verankerungselementes in einer Seitenansicht und in einer dazu senkrechten Ansicht;
- Fig. 2: einen Teil des Verankerungselementes einer ersten Ausführungsform mit einem eingelegten Stab, in geschnittener Darstellung;
- Fig. 3: einen Schnitt entlang der Linie III-III in Fig. 2;
- Fig. 4: einen Schnitt durch eine zweite Ausführungsform;
- Fig. 5: einen Schnitt entlang der Linie V-V in Fig. 4;
- Fig. 6: einen Schnitt durch eine dritte Ausführungsform;
- Fig. 7: einen Schnitt entlang der Linie VII-VII in Fig. 6;
- Fig. 8: einen Schnitt durch eine weitere abgewandelte Ausführungsform;
- Fig. 8a: ein Detail auf Fig. 8 in vergrößerter Darstellung;
- Fig. 9: einen Schnitt entlang der Linie IX-IX in Fig. 8;
- Fig. 10: eine Explosionsdarstellung der in Fig. 8 gezeigten Ausführungsform;
- Fig. 11: einen Schnitt durch eine weitere abgewandelte Ausführungsform;
- Fig. 12: einen Schnitt entlang der Linie XII-XII in Fig. 11;
- Fig. 13: eine explosionsartige Darstellung der in den Fig. 11 und 12 gezeigten Ausführungsform;
- Fig. 14: einen Schnitt eines Teiles aus Fig. 13 entlang der Linie XIV-XIV in Fig. 13;
- Fig. 15: eine Explosionsdarstellung einer weiteren Ausführungsform;
- Fig. 15a: einen Schnitt entlang der Linie XV-XV im Fig. 15;
- Fig. 16: die Ausführungsform in zusammengesetztem Zustand;
- Fig. 17: einen Schnitt entlang der Linie XVII-XVII in Fig. 16;
- Fig. 18: einen Schnitt durch eine weitere Ausführungsform;
- Fig. 19: die Ausführungsform in Explosionsdarstellung;
- Fig. 20: eine Detaildarstellung;
- Fig. 20a: einen Schnitt entlang der Linie XX-XX in Fig. 20;
- Fig. 21: eine teilgeschnittene Ansicht eines Justierwerkzeuges; und
- Fig. 22: eine Schnittansicht des Verankerungselementes mit eingelegtem Stab und eingesetztem Justierwerkzeug.

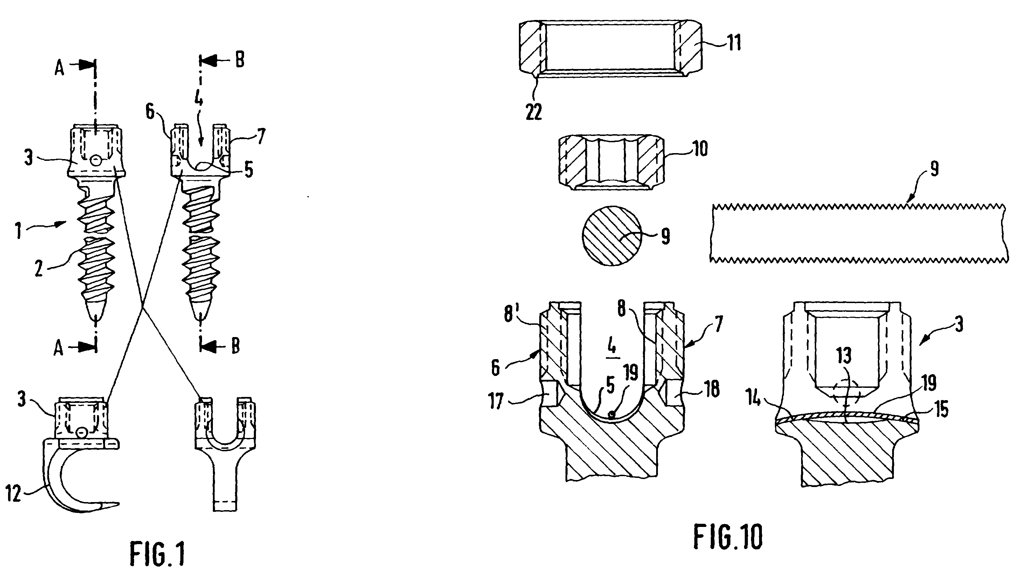

Das in Fig. 1 gezeigte Verankerungselement umfaßt eine in eine Wirbelsäule einzubringende Schraube 1 mit einem Gewindeschaft 2 und einem Kopf 3. Der Kopf weist eine zur Mittenachse des Gewindeschaftes 2 symmetrisch angeordnete U-förmige Ausnehmung 4 auf, deren Grund 5 sich auf der den Gewindeschaft aufweisenden Seite befindet. Die Seitenwandung des die U-förmige Ausnehmung 4 begrenzenden Kopfes wird durch seitliche freie Schenkel 6, 7 gebildet. Im Inneren des durch die U-förmige Ausnehmung 4 gebildeten Kanales ist eine konzentrisch zur Mittenachse des Gewindeschaftes 2 ausgebildete Bohrung mit einem Innengewinde 8 vorgesehen. Der Kopf 3 selbst ist zylindrisch ausgebildet und weist ein Außengewinde 8' auf.

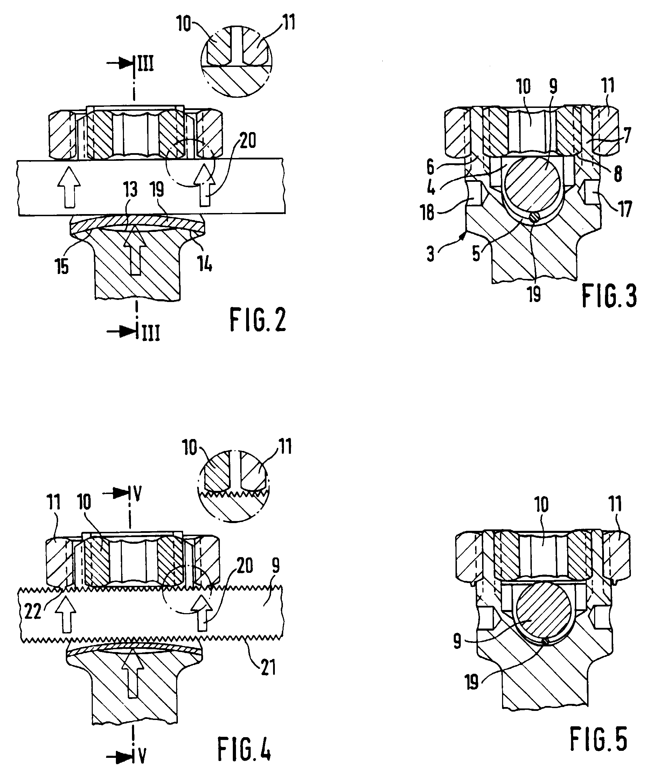

Damit mit dieser Schraube ein Stab 9 verankert werden kann, ist ein als Gewindeschraube ausgebildetes Fixierelement 10 vorgesehen. Das Fixierelement 10 weist ein mit dem Innengewinde 8 zusammenwirkendes Außengewinde zum Einschrauben in die U-förmige Ausnehmung 4 auf. Das Fixierelement 10 weist eine konzentrische Ausnehmung zum Eingreifen mit einem Imbusschlüssel auf. Ferner ist ein die beiden U-förmigen Schenkel 6, 7 von außen umfassendes Element in Form einer Überwurfmutter 11 vorgesehen, deren Gewinde mit dem Außengewinde 8' zusammenwirkt. Die Drehrichtung des Innengewindes 8 und des zugehörigen Fixierelementes 10 einerseits und des Außengewindes 8' und der Überwurfmutter andererseits sind bevorzugt entgegengesetzt gerichtet. Wie z.B. aus Fig. 3 ersichtlich ist, weist der Grund 5 der U-förmigen Ausnehmung einen Radius auf, der nur so viel größer ist, als der Radius des aufzunehmenden Stabes 9, daß der Stab in die U-förmige Ausnehmung leicht einsetzbar bzw. leicht aus dieser herausnehmbar ist. Das Innengewinde 8 und das Außengewinde 8' erstrecken sich jeweils so weit nach unten, also in Richtung des Grundes 5, daß die Projektion auf die Symmetrieachse einen Abstand vom Grund 5 aufweist, der kleiner ist als der Durchmesser des aufzunehmenden Stabes 9.

Wie aus Fig. 1 ersichtlich ist, kann das Verankerungselement alternativ anstelle des Gewindeschaftes 2 einen Haken 12 aufweisen. Dieser dient in gleicher Weise zur Verbindung mit einem Wirbelsäulenelement, wobei der Haken in einen Wirbelbogen eingebracht wird.

Bei dem in den Fig. 2 und 3 gezeigten ersten Ausführungsbeispiel ist der Grund der jeweiligen U-förmigen Ausnehmung gewölbt ausgebildet. Die Wölbung weist einen inneren ersten konkaven Abschnitt 13 und zwei zur Mittenachse der Schraube symmetrisch angeordnete nach oben erhaben hervorstehende gewölbte zweite Bereiche 14, 15 auf. Der Abstand der jeweils höchsten Stelle der zweiten Bereiche von der Mittenachse der Schraube ist im wesentlichen gleich dem Abstand der Mitte 16 zwischen dem Radius des Innengewindes 8 und dem Radius des Außengewindes 8' von der Symmetrieachse der Schraube. Wie am besten aus Fig. 3 ersichtlich ist, weist der Kopf 3 zwei gegenseitig um 180° versetzte Ausnehmungen 17, 18 auf, die im wesentlichen jeweils um 90° gegen die U-förmige Ausnehmung 4 versetzt sind und die zum Eingreifen mittels eines Handhabungswerkzeuges, beispielsweise einer Greifzange, dienen.

Wie am besten aus Fig. 2 ersichtlich ist, ist am Grund 5 des durch die Ausnehmung 4 gebildeten Kanales ein Federelement 19 in Form einer Stabfeder eingelassen. Zu diesem Zweck weist der Grund in den seitlichen erhabenen zweiten Bereichen jeweils eine Rille auf, in der der Randabschnitt des Federelementes 19 aufgenommen wird. An einer der beiden Seiten ist das Federelement durch Laserpunktschweißen angeheftet. Wie aus Fig. 2 ersichtlich ist, ist die zur Kanalöffnung hin gesehen konvexe Wölbung des Federelementes 19 so gewählt, daß sie ohne zusätzliche Krafteinwirkung auf den Stab 9 den Stab von dem Grund weg in einen Abstand von den erhabenen zweiten Bereichen anhebt.

In dem oben beschriebenen Ausführungsbeispiel ist das Federelement 19 als Stabfeder ausgebildet. Die Feder kann auch eine andere Form haben. Insbesondere kann es sich um eine Blattfeder handeln, die in entsprechender Weise eingelegt ist. Es kann auch eine zur Symmetrieachse der Schraube ausgerichtete Druckfeder sein. Bei Verwendung einer Blattfeder bzw. einer Stabfeder sind diese vorzugsweise jeweils so ausgerichtet, daß sie mit der Längs-Mittenachse des Kanales zusammenfallen.

Im Betrieb wird zunächst der Stab 9 in den durch die Ausnehmung 4 gebildeten Kanal eingelegt. Die Überwurfmutter 11 wird lose so aufgeschraubt, daß sie mit geringer Klemmwirkung den Stab 9 in seiner gewählten Position hält, ohne daß das Federelement 19 bereits eingedrückt werden würde. Das Fixierelement 10 wird so eingeschraubt, daß es ebenfalls in eine Art Reibkontakt mit dem Stab gelangt. Durch das Zusammenwirken insbesondere von Überwurfmutter 11 und Federelement 19 wird der Stab 9 in seiner Position geklemmt gehalten. In jedem Wirbelsäulensegment ist ein entsprechendes Verankerungselement angeordnet und mit dem Stab 9 in der zunächst vom Operateur gewählten Position verklemmt.

In einem nächsten Abschnitt erfolgt Segment für Segment eine Feineinstellung, bei dem die Angriffsposition der einzelnen Verankerungselemente an dem Stab relativ zur Längsachse des Stabes einzustellen ist. Zu diesem Zweck wird an dem entsprechenden Verankerungselement an den beiden gegenüberliegenden Seiten des Stabes angrenzend an die Überwurfmutter 11 ein Druck auf den Stab 9 in einer Richtung entgegen der in Fig. 2 gezeigten Pfeile 20 ausgeübt, so daß der Stab von der Überwurfmutter 11 und dem Fixierelement 10 abgehoben und somit leicht in einer Richtung parallel zur Achse des Stabes in eine der Feinjustierung entsprechende Position verschiebbar ist. Anschließend wird die Stange wieder losggelassen und in der in Fig. 2 gezeigten Position geklemmt. Dieser Justiervorgang kann gewünschtenfalls mehrfach wiederholt werden. Anschließend wird die Überwurfmutter 11 so fest angezogen, daß der Stab 9 das Federelement 19 vollständing auf den Grund drückt und der Stab 9 dann zwischen Überwurfmutter 11 und Grund kraftschlüssig voll eingespannt ist. Nun wird das Fixierelement 10 in Richtung des Stabes geschraubt, bis eine vorgewünschte Haltekraft ausgeübt wird. Dadurch wird erreicht, daß die Überwurfmutter 11 in ihrer Position verklemmt wird und sich im zukünftigen Betrieb nicht lockert.

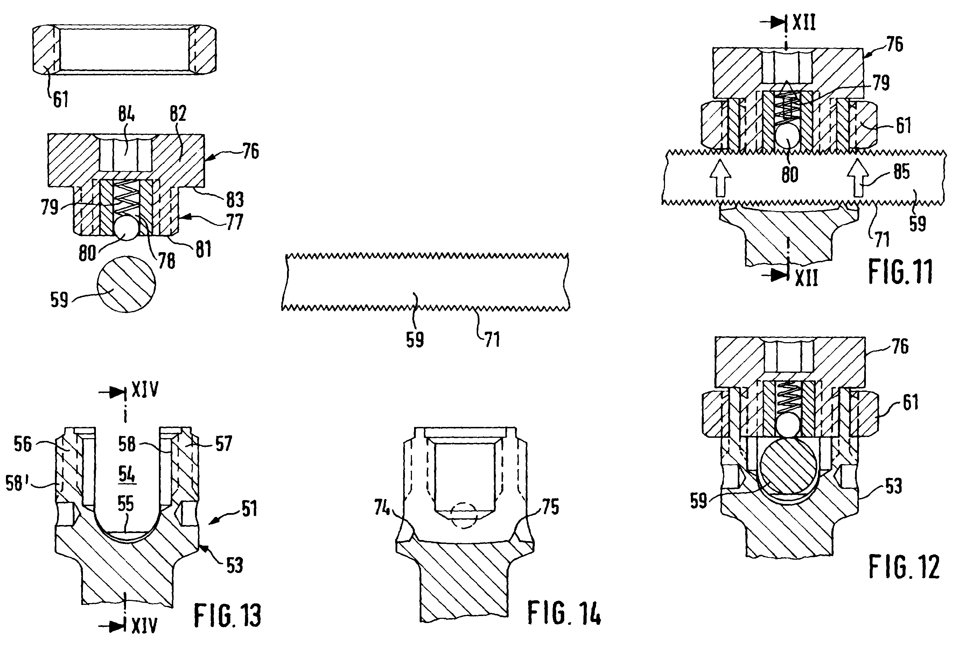

Die in den Fig. 4 und 5 gezeigte Ausführungsform unterscheidet sich von der vorher beschriebenen durch folgende Merkmale: Die Oberfläche des Stabes 9 ist als Gewinde 21 ausgebildet. Ferner weist der dem Stab 9 zugewandte Rand der Überwurfmutter 11 einen am besten aus Fig. 10 ersichtlichen hervorstehenden Ring 22 auf. Dieser ist in seinen Abmessungen in Abhängigkeit von der Art des Gewindes 21 so gewählt, daß er bei der in Fig. 4 gezeigten auf den Stab aufgeschraubten Position in das Gewinde eingreift.

Im Betrieb werden bei dieser zweiten Ausführungsform zunächst wiederum die Überwurfmutter 11 und das Fixierelement 10 in die in Fig. 4 gezeigte Lage gedreht, in der die Relativlage zwischen Verankerungselement und Stab durch das Verklemmen unterstützt durch den Eingriff des Ringes 22 in das Gewinde 21 festgelegt ist. Zur anschließenden Feinjustierung wird der Stab 9 wie oben beschrieben entgegen der Richtung der Pfeile 20 und damit entgegen der Federvorspannung nach unten gedrückt und damit der Eingriff zwischen Ring 22 und Gewinde bzw. zwischen Überwurfmutter und Fixierelement einerseits und Stab 9 andererseits gelöst. In dieser niedergedrückten Stellung wird der Stab entsprechend der gewünschten Justierung des Segmentes verschoben. In der neuen gewünschten Position wird der auf den Stab wirkende Druck aufgehoben, so daß die in Fig. 4 gezeigte Arretierungsposition wieder eingestellt wird. Zur endgültigen Fixierung werden zunächst die Überwurfmutter und das Fixierelement wieder so weit in Richtung des Grundes 5 gedreht, bis jedes der beiden Teile eine gewünschte Haltekraft auf den Stab 9 ausübt und die Überwurfmutter 11 verriegelt ist.

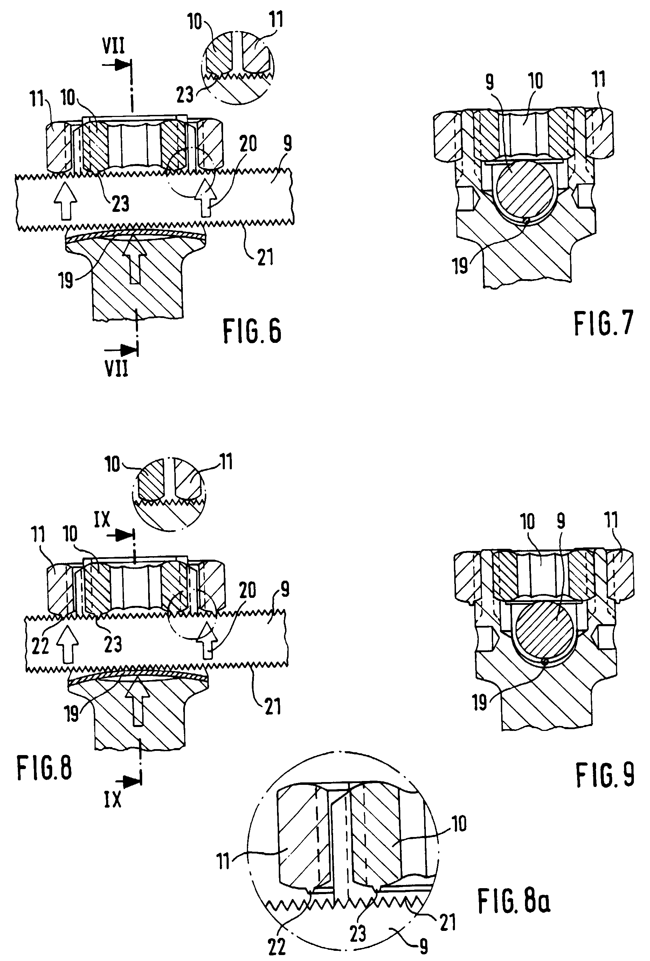

Die in Fig. 6 und 7 gezeigte Ausführungsform unterscheidet sich von der zuvor beschriebenen zweiten Ausführungsform dadurch, daß die Überwurfmutter in der gleichen Weise wie bei dem ersten Ausführungsbeispiel ausgebildet ist und das Fixierelement 10 einen dem Ring 22 entsprechenden Ring 23 aufweist, der bei Inkontaktkommen des Fixierelementes mit dem Stab in das Gewinde eingreift und in Kooperation mit der durch das Federelement 19 bewirkten Verklemmung eine axiale Verschiebung des Stabes 9 relativ zum Verankerungselement verhindert. Die Feinverstellung und die endgültige Arretierung erfolgen in gleicher Weise wie bei dem zweiten Ausführungsbeispiel.

Das in den Fig. 8 bis 10 gezeigte vierte Ausführungsbeispiel unterscheidet sich von dem zweiten bzw. dritten Ausführungsbeispiel dadurch, daß die Überwurfmutter wie bei dem zweiten Ausführungsbeispiel den Ring 22 und das Fixierelement wie bei dem dritten Ausführungsbeispiel den Ring 23 aufweisen. Im Betrieb erfolgt die Verklemmung für die Voreinstellung, die Feinjustierung durch Ausüben des Druckes auf den Stab 9 entgegen der Richtung der Pfeile 20 und das anschließende endgültige Verriegeln in der gleichen Weise wie bei den vorherigen Ausführungsbeispielen. Die beiden Ringe 22 und 23 greifen im Zustand der durch die Federkraft des Federelementes 19 bewirkten Klemmstellung in das Gewinde 21 ein und tragen somit zum Verriegeln des Stabes gegen eine axiale Verschiebung relativ zum Verankerungselement bei.

Die in den Fig. 11 bis 14 gezeigte weitere Ausführungsform unterscheidet sich von den zuvor beschriebenen Ausführungsformen dadurch, daß das Federelement nicht auf dem Grund der den Kanal bildenden Ausnehmung 4 sondern auf der in axialer Richtung gesehen gegenüberliegenden Seite vorgesehen ist. Das als Schraube 51 bzw. Haken ausgebildete Verankerungselement weist einen Kopf 53 mit einer einen Kanal bildenden Ausnehmung 54 auf. Der Kanal wird begrenzt von dem Grund des Kanales 55 und seitlichen Schenkeln 56, 57. In gleicher Weise wie bei den vorhergehenden Ausführungsformen sind ein Innengewinde 58 und ein Außengewinde 58' vorgesehen. Der Kanal dient zur Aufnahme eines Stabes 59, der die gleiche Form aufweist wie der Stab 9. Es ist eine Überwurfmutter 61 vorgesehen, die den gleichen Aufbau wie die Überwurfmutter 11 bei dem ersten Ausführungsbeispiel besitzt.

Abweichend von den bisher beschriebenen Ausführungebeispielen weist der am besten aus Fig. 14 ersichtliche Grund 55 des Kanales eine sich in wesentlichen senkrecht zur Schraubenachse erstreckende gerade Form auf. An den beiden seitlichen Abschnitten des Kanalbodens sind in Richtung des offenen Endes des Kanales nach oben hervorstehende Ansätze 74, 75 vorgesehen. Diese liegen vorzugsweise in der Symmetrieebene des Kanales. Ihre Form ist in Abhängigkeit von den Gewinde 71 des Stabes 59 so gewählt, daß die Ansätze bei Ineingriffbringen mit den Stab 59 in das Gewinde eingreifen können. Der Abstand der beiden Ansätze 74, 75 von der Mittenachse der Schraube ist so gewählt, daß jeder Ansatz in etwa in der Mitte zwischen den Radius für das Innengewinde 58 und dem Radius für das Außengewinde 58' liegt.

Es ist ein Justierkopf 76 vorgesehen. Dieser weist einen Gewindeabschnitt 77 auf, dessen Außengewinde so gewählt ist, daß es mit dem Innengewinde 58 zusammenpaßt. Der Gewindeabschnitt 77 weist auf seiner im Betrieb dem Kanalgrund zugewandten Seite eine konzentrische Sackbohrung 78 auf, in der eine Druckfeder 79 aufgenommen ist. An der den Grund der Sackbohrung abgewandten Seite ist ein Kugelelement 80 vorgesehen. Die Druckfeder 79 ist so bemessen, daß sie das Kugelelement 80 in der in Fig. 13 gezeigten Weise um ein vorbestimmtes Maß über die Stirnseite 81 des Gewindeabschnittes hervorstehen läßt.

Auf der der Stirnseite 81 abgewandten Seite weist der Justierkopf einen Zylinderabschnitt 82 auf, dessen Durchmesser größer ist als der Durchmesser des Gewindeabschnittes, so daß auf der den Gewindeabschnitt zugewandten Seite ein sich in radialer Richtung erstreckender Anschlagsrand 83 ergibt. Auf der dem Gewindeabschnitt 77 abgewandten Oberfläche weist der Zylinderabschnitt 82 eine konzentrische Sechskantbohrung 84 zum Einführen eines Schraubelementes auf.

Im Betrieb wird zunächst in der in Fig. 11 gezeigten Weise der Stab 59 in den Kanal eingelegt. Es wird die Überwurfmutter 61 lose aufgeschraubt und dann wird der Justierkopf bis zu seinem Anschlag aufgeschraubt. Die in axialer Richtung sich erstreckende Länge des Gewindeabschnittes 77 ist in Abhängigkeit von der Länge der Schenkel 56, 57 und des Durchmessers des Stabes 59 so gewählt, daß bei Einschrauben bis zum Anschlag durch Zusammenwirken von Druckfeder 79 und Kugelelement 80 die Vorspannung auf den Stab 59 so groß ist, daß der Stab 59 und die Ansätze 74, 75 so fest in Eingriff sind, daß sich der Stab 59 nicht in seiner Relativlage zum Verankerungselement verschieben läßt. Zu der oben beschriebenen Feinjustierung wird der Stab 59 in Richtung der Pfeile 85 zu dem Justierkopf hin so weit bewegt, bis der Eingriff zwischen den Ansätzen 74, 75 und dem Gewinde 71 gelöst wird. In dieser Position kann der Stab 59 nunmehr relativ zum Verankerungselement seitlich in eine der Feinjustierung entsprechende Position verschoben werden. Nach dem Verschieben wird der Stab losgelassen, wodurch er aufgrund der Vorspannung der Druckfeder 79 wieder in die in Fig. 11 gezeigte Arretierstellung mit in Eingriff der Ansätze 74, 75 mit dem Gewinde 71 gelangt.

Nach erfolgter Feinjustierung wird die Überwurfmutter 11 so weit in Richtung des Grundes gedreht, bis eine gewünschte Haltekraft auf den Stab 59 ausgeübt wird. Grundsätzlich ist es möglich, den Justierkopf in seiner Lage zu belassen. Vorzugsweise ist das Verankerungselement jedoch so ausgebildet, daß zusätzlich zu dem Justierkopf 76 eine dem Fixierelement 10 der ersten Ausführungsform entsprechende Schraube vorgesehen ist. Nach der Justierung wird der Justierkopf herausgeschraubt und es wird an seiner Stelle das Fixierelement 10 so weit eingeschraubt, daß eine gewünschte Haltekraft auf den Stab 59 ausgeübt wird, wodurch vor allem eine Verklemmung zwischen der Überwurfmutter 61 und den Schenkeln 56, 57 erfolgt, so daß sich die Überwurfmutter nicht von selbst lösen kann. Bei den oben beschriebenen Ausführungsbeispielen ist der Stab 9 bzw. 59 entweder als glatter Stab oder als Gewindestab ausgebildet. Anstelle des Gewindestabes kann der Stab auch eine als Kreuzgewinde ausgebildete Oberfläche oder eine Oberfläche mit sich in radialer Richtung erstreckenden Rillen aufweisen. Der Stab 9 bzw. 59 kann auch so ausgebildet sein, daß er auf seiner mit den Ringen 22, 23 bzw. Ansätzen 74, 75 zusammenwirkenden Seite eine zahnstangenartige Ausbildung aufweist.

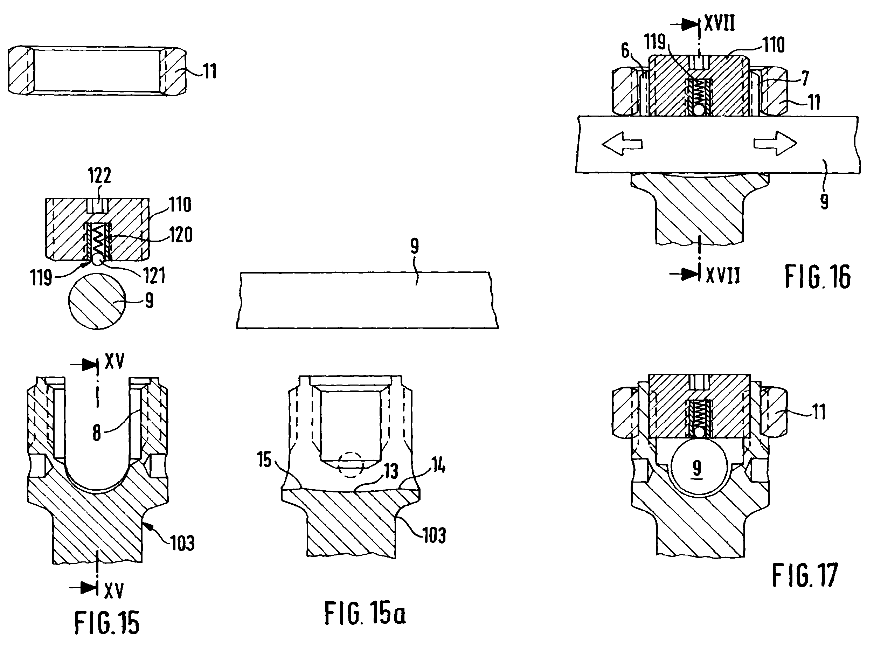

Die in den Fig. 15 bis 17 gezeigte Ausführungsform weist ein Verankerungselement mit einem Kopf 103 auf, der dem Kopf 3 des ersten Ausführungsbeispieles entspricht mit der Abweichung, daß der Kopf 103 kein Federelement aufweist. Der Stab 9 entspricht dem des ersten Ausführungsbeispieles, gleichermaßen die Überwurfmutter 11. Zur Fixierung ist ein Fixierelement 110 vorgesehen, welches als Zylinderelement ausgebildet ist und auf seiner Außenseite ein Gewinde aufweist, welches mit dem Innengewinde des Kopfes 3 zusammenpaßt. Auf seiner dem Kanalgrund zugewandten Seite weist das Fixierelement 110 ein Federelement 119 auf. Dieses umfaßt eine Sackbohrung, in der eine Druckfeder 120 vorgesehen ist, die ausgangsseitig ein Kugelelement 121 trägt. Die Bemessung der Feder ist so gewählt, daß die Kugel um ein vorbestimmtes Ausmaß über die angrenzende Stirnfläche des Fixierelementes hervorsteht. Auf der entgegengesetzten Stirnfläche weist das Fixierelement 110 eine koaxiale Sechskantbohrung 122 zur Aufnahme eines Schraubendrehers auf.

Im Betrieb wird zunächst der Stab 9 in den Kanal eingelegt. Daran anschließend wird die Überwurfmutter 11 so weit aufgedreht, daß noch ein Spiel zwischen Überwurfmutter 11 und Stab 9 verbleibt. Dann wird das Fixierelement 110 so weit eingeschraubt, daß noch ein Spiel zwischen der dem Stab 9 zugewandten Stirnfläche des Fixierelementes und dem Stab verbleibt, daß andererseits aber die Kugel des Federelementes 119 mit dem Stab 9 derart in Kontakt ist, daß das Federelement 119 auf den Stab 9 eine solche Kraft ausübt, daß die Auflage des Stabes 9 auf den erhabenen Bereichen 14, 15 einerseits und das Einwirken des Federelementes 119 auf der gegenüberliegenden Seite andererseits eine ausreichende Verklemmung des Stabes 9 gegen eine Verschiebung des Stabes relativ zum Verankerungselement sichert.

Zum Zwecke einer Verschiebung im Zusammenhang mit einer Feinjustierung wird der Stab 9 ohne Verstellen des Fixierelementes in die eine oder andere Richtung in der Weise gezogen, daß durch das Aufbringen der Zugkraft die Klemmkraft überwunden wird. Nach Beendigung der Feinjustierung wird zunächst die Überwurfmutter 11 so weit festgezogen, daß die gewünschte Haltekraft erreicht ist. Anschließend wird das Fixierelement 110 um ein vorbestimmtes Maß festgezogen, so daß eine ausreichende Verklemmung zwischen den Schenkeln 6 und 7 einerseits und der Überwurfmutter andererseits erzielt und damit ein unbeabsichtigtes Lösen derselben vermieden wird.

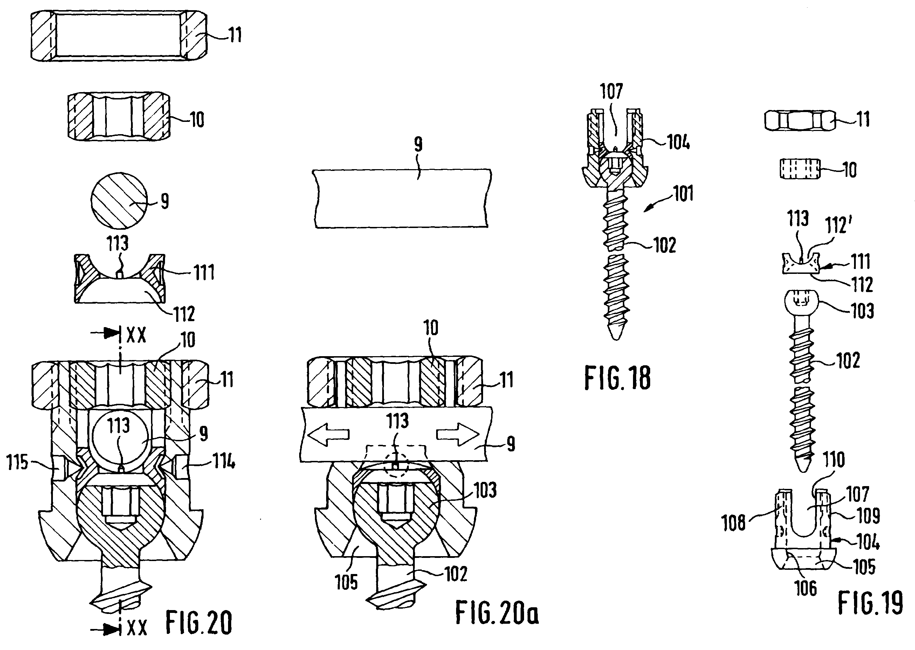

Die in den Fig. 19 bis 20a gezeigte Ausführungsform unterscheidet sich von den vorhergehenden insbesondere dadurch, daß die Schraube als eine sogenannte Polyaxialschraube ausgebildet ist, bei der der den Stab aufnehmende Kopf und der zugehörige Gewindeschaft gegeneinander verschwenkbar ausgebildet sind. Soweit die Elemente mit denjenigen der vorher beschriebenen Ausführungsformen übereinstimmen, sind die gleichen Bezugszeichen verwendet.

Die Schraube 101 weist einen Gewindeschaft 102 und einen einen kugelsegmentförmigen Abschnitt besitzenden Kopf 103 auf. Ferner ist ein zylindrisches Aufnahmeteil 104 für die Aufnahme des Kopfes 103 des Schraubenelementes vorgesehen. Dieses weist an seinem einen Ende eine erste Bohrung 105 zum Hindurchführen des Gewindeabschnittes 102 auf. Daran angrenzend ist innen ein hohlkugelförmiger Abschnitt 106 vorgesehen zum Anlegen des kugelförmigen Kopfes 103. Ferner ist eine auf der der ersten Bohrung 105 gegenüberliegenden Seite offene zweite Bohrung 107 zum Einführen des Gewindeschaftteiles 102 mit Kopf 103 vorhanden. Das Zylinderteil weist eine zur Mittenachse des Gewindeschaftes symmetrisch angeordnete U-förmige Ausnehmung auf. Die Seitenwandung des die U-förmige Ausnehmung begrenzenden Kopfes wird wie beim ersten Ausführungsbeispiel durch seitliche freie Schenkel 108, 109 gebildet. Im Inneren des durch die U-förmige Ausnehmung gebildeten Kanales ist eine konzentrisch zur Mittenachse des Gewindeschaftes ausgebildete Bohrung mit einem Innengewinde 110 vorgesehen. Der Kopf 104 weist auf seiner Außenseite ein Außengewinde auf. Es ist ein auf den Kopf 103 einwirkendes Druckelement 111 vorgesehen, welches so ausgebildet ist, daß es auf seiner dem Kopf 103 zugewandten Seite eine sphärische Ansenkung 112 aufweist, deren Radius im wesentlichen gleich dem Radius des kugelsegmentförmigen Abschnittes des Kopfes 103 ist. Der Außendurchmesser des Druckelementes ist so gewählt, daß das Druckelement in der zylinderförmigen Ausnehmungen hin- und herschiebbar ist. Auf seiner dem Kopf 103 abgewandten Seite weist die Oberfläche eine sich senkrecht zur Schraubenachse erstreckende zylinderabschnittförmige Ausnehmung 112' auf. Auf dem Grund dieser Ausnehmung ist analog zu dem ersten Ausführungsbeispiel ein dem Federelement 19 entsprechendes Federelement 113 vorgesehen. Bei der Fertigungsmontage sind die Teile 102, 103 und 111 in der in Fig. 18 gezeigten Weise montiert. Damit die Achse der zylindrischen Ausnehmung 112 genau in der Symmetrieebene des U-förmigen Kanales liegt, wird das Druckelement 111 bei der Montage in der am besten aus der Fig. 20 ersichtlichen Weise durch ein Kröpfen über Bohrungen 114, 115 in seiner Lage fixiert.

Ferner sind wie beim ersten Ausführungsbeispiel ein Fixierelement 10 und eine Überwurfmutter 11 vorgesehen.

Im Betrieb wird in das in der in Fig. 18 gezeigten Weise vormontierte Verankerungselement der Stab 9 in den U-förmigen Kanal eingelegt. Es erfolgt dann die erste Fixierung, Feinjustierung und endgültige Arretierung in der gleichen Weise wie beim ersten Ausführungsbeispiel unter Verwendung des Fixierelementes 10 und der Überwurfmutter 11.

In dem zuvor beschriebenen Ausführungebeispiel ist der bewegliche Schraubenkopf im Zusammenhang mit dem zuvor beschriebenen ersten Ausführungsbeispiel beschrieben. Anstelle des glatten Stabes 9 kann ein Stab mit den strukturierten Oberflächen entsprechend der oben beschriebenen Beispiele verwendet werden. Das Fixierelement 10 bzw. die Überwurfmutter 11 können wie bei den oben beschriebenen Beispielen Ringe 22 bzw. 23 aufweisen. Schließlich kann statt des Federelementes 113 ein dem Justierkopf 76 entsprechender Justierkopf oder ein Fixierelement 110 mit Federelement verwendet werden.

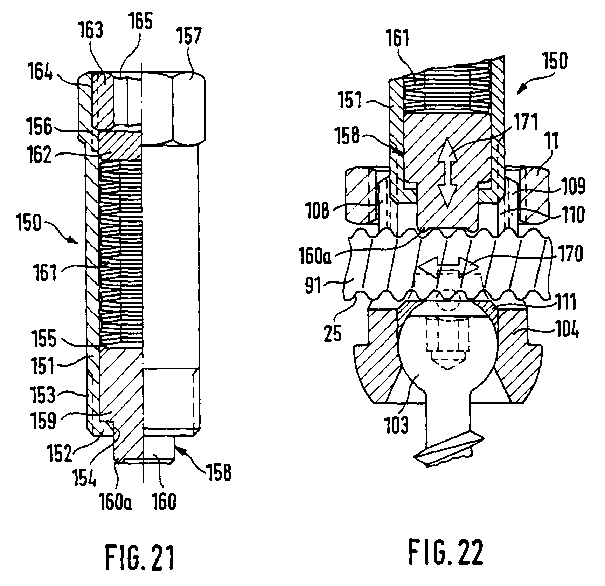

Die Oberfläche eines Stabes 91 kann beispielsweise eine schraubenförmige Wellung bzw. ein Rundgewinde 25, wie insbesondere aus Fig. 22 ersichtlich ist, aufweisen.

Zum Einstellen der Position des Aufnahmeteiles 104 relativ zu dem Stab und zum Fixieren des Kopfes 103 der Polyaxialschraube in dem Aufnahmeteil 104 ist ein in Fig. 21 gezeigtes Justierwerkzeug 150 vorgesehen. Das Justierwerkzeug weist ein Gehäuse mit einem zylinderförmigen Mantel 151 und einer Stirnseite 152 auf. In einem an die Stirnseite 152 angrenzenden Bereich des Mantels 151 ist ein Außengewinde 153 vorgesehen, welches dem Innengewinde 110 des Aufnahmeteiles 104 entspricht. In der Stirnseite 152 ist eine kreisförmige Öffnung 154 vorgesehen. Angrenzend an die Öffnung 154 ist im Inneren des Gehäuses eine koaxiale Bohrung 155 vorgesehen, deren Durchmesser größer als der Durchmesser der Öffnung 154 ist. Die Bohrung 155 erstreckt sich bis an das der Stirnseite 152 gegenüberliegende Ende des Mantels 151, so daß das Gehäuse an diesem Ende offen ist. An diesem der Stirnseite 152 abgewandten Ende des Mantels weist der Mantel ein Innengewinde 156 auf. Das Gehäuse weist ferner in dem das Innengewinde 156 aufweisenden Bereich des Mantels einen Abschnitt 57 mit einem in einer Ebene senkrecht zur Zylinderachse des Mantels 151 sechskantförmigen Querschnitt zum Ineingriffgelangen mit einem Schraubenschlüssel auf.

Das Justierwerkzeug weist ferner ein in dem Gehäuse gehaltenes Druckteil 158 mit einem ersten zylinderförmigen Abschnitt 159 und einem daran angrenzenden zweiten zylinderförmigen Abschnitt 160 auf, wobei der Außendurchmesser des ersten zylinderförmigen Abschnittes etwas geringer als der Durchmesser der Bohrung 155 ist und der Außendurchmesser des zweiten zylinderförmigen Abschnittes 160 etwas geringer als der Durchmesser der Öffnung 154 ist, so daß das Druckteil 158 in axialer Richtung in der Bohrung 155 bis zu einem durch die Stirnseite gebildeten Anschlag verschiebbar ist, wobei das Druckteil 158 dann mit seinem zweiten zylinderförmigen Abschnitt 160 aus der Öffnung 154 hervorsteht. Das Justierwerkzeug weist ferner eine im Inneren der Bohrung 155 angeordnete und auf das Druckteil 158 einwirkende Druckfeder in Form von übereinander geschichteten Tellerfedern 161 auf, deren Außendurchmesser etwas geringer als der Durchmesser der Bohrung 155 ist. Ferner ist in der Bohrung 155 auf der dem Druckteil 158 abgewandten Seite der Tellerfedern 161 ein auf diese einwirkendes zylinderförmiges Druckelement 162 vorgesehen, dessen Außendurchmesser etwas geringer als der Durchmesser der Bohrung 155 ist, so daß dieses in der Bohrung leicht in axialer Richtung verschiebbar ist. Das Druckelement 162 wird durch eine in der Bohrung 155 angeordnete und auf diese einwirkende Gewindeschraube 163 mit einem Außengewinde 164, die in das Innengewinde 156 des Mantels geschraubt ist, gegen die Tellerfedern 161 gedrückt. Die Gewindeschraube 163 weist ferner eine zentrale sechskantförmige Ausnehmung 165 zum Ineingriffgelangen mit einem Imbusschlüssel auf.

An seiner aus der Öffnung 154 herausstehenden Stirnseite weist der zweite zylinderförmige Abschnitt 160 des Druckteiles 158 einen ringförmigen Vorsprung 160a auf, wobei der Ring durch eine koaxiale kreisförmige Ausnehmung in der Stirnseite des Abschnittes 160 gebildet ist. Die Kanten des ringförmigen Vorsprunges 160a sind abgerundet. Der Durchmesser des ringförmigen Vorsprunges 160a entspricht einem ganzzahligen Vielfachen der Teilung des Rundgewindes 25 des aufzunehmenden Stabes 91 und die Höhe des ringförmigen Vorsprungs ist kleiner als die Gangtiefe des Rundgewindes 25, so daß jeweils gegenüberliegende Bereiche des ringförmigen Vorsprunges 160a in die Gewindegänge des Stabes 91 eingreifen.

Im Betrieb erfolgt die Feineinstellung der Positionen der einzelnen Verankerungselemente auf dem Stab Segment für Segment.

Dazu wird in ein in der in Fig. 22 gezeigten Weise vormontierte Verankerungselement mit eingelegtem Stab 91 die Überwurfmutter 11 lose aufgeschraubt und dann das Justierwerkzeug so weit in das Innengewinde 110 der Schenkel eingeschraubt, bis der Stab 91 durch das durch die Tellerfedern 161 vorgespannte Druckteil 158 fest auf das auf dem Schraubenkopf 103 aufliegende Druckelement 111 gedrückt wird. Dadurch wird eine Bewegung des Schraubenkopfes 103 relativ zu dem Aufnahmeteil 104 blockiert. Gleichzeitig greift der ringförmige Vorsprung 160a in die Gewindegänge des Rundgewindes 25 des Stabes 91 ein, so daß ein Verschieben des Stabes in den in Fig. 22 durch die Pfeile 170 gezeigten Richtungen parallel zur Stabachse ohne Einwirken äußerer Kräfte nicht möglich ist. Die Vorspannung des Druckteiles 158 kann dabei durch Zusammendrücken der Tellerfedern 161 mittels des Druckelementes 62 über die Gewindeschraube 163 geeignet eingestellt werden.

Zur Feinjustierung der Stellung des Aufnahmeteiles 104 relativ zu dem Stab 91 kann dann der Stab 91 durch manuelles Ziehen bzw. Schieben in den durch die Pfeile 170 in Fig. 22 gezeigten Richtungen parallel zur Stabachse in seiner Lage verändert werden, wobei das Justierwerkzeug wie eine Ratsche wirkt. Dabei gelangen die entsprechenden Bereiche des ringförmigen Vorsprunges 160a des Druckteiles 158 außer Eingriff mit dem Rundgewinde 25 des Stabes 91, wodurch das Druckteil 158 in einer Richtung entgegen seiner Vorspannungsrichtung, wie durch den Pfeil 171 in Fig. 22 gezeigt ist, bewegt wird, bis der ringförmige Vorsprung 160a in den nächsten Gewindegang einrastet und sich das Druckteil 158 wieder in seiner vorgespannten Stellung befindet. Die aufzuwendende Kraft zur Einstellung der relativen Lage von Stab 91 und Aufnahmeteil 104 hängt dabei von der Vorspannung des Druckteiles 158 ab.

Das Justierwerkzeug bietet dabei den Vorteil, daß eine Feineinstellung in Einheiten der Teilung des Gewindes 25 des Stabes 91 möglich ist. Da das jeweilige Einrasten des ringförmigen Vorsprunges 160a in die Gänge des Rundgewindes leicht akustisch zu vernehmen ist, kann die entsprechende Feineinstellung leicht durchgeführt werden.

## Patentansprüche

1. Verankerungselement mit einen zur Knochenverankerung bestimmten Schaft (2, 12) und einem mit einem Stab (9, 59) verbindbaren Kopf (3, 53) mit einen im wesentlichen U-förmigen Querschnitt, der an seinen Grund (5, 55) mit dem Schaft (2, 12) verbunden ist und zwei einen Kanal zur Aufnahme des Stabes (9, 59) bildende freie Schenkel (6, 7; 56, 57) hat,
wobei die Schenkel ein Innengewinde (8, 58) und ein Außengewinde (8', 58') aufweisen,
und mit einem der Schenkel (6, 7; 56, 57) von außen umfassenden, ein mit den Außengewinde zusammenwirkendes Innengewinde aufweisenden Mutternelement (11, 61) und einem ein mit dem Innengewinde der Schenkel zusammenwirkendes Gewinde aufweisendes Fixierelement (10),
dadurch gekennzeichnet, daß
ein mit seiner Federkraft auf den aufzunehmenden Stab (9, 59) einwirkendes Feder_ element (19, 79) vorgesehen ist.

2. Verankerungselement nach Anspruch 1,
dadurch gekennzeichnet, daß
am Kanalgrund ein Federelement (19, 79) vorgesehen ist.

3. Verankerungselement nach Anspruch 1,
dadurch gekennzeichnet, daß
das Federelement (19, 79) in eine Richtung parallel zur Schaftachse vorgespannt ist.

4. Verankerungselement nach Anspruch 3,
dadurch gekennzeichnet, daß
das Federelement (9) zum offenen Ende des Kanales hin vorgespannt ist.

5. Verankerungselement nach einem der Ansprüche 1 bis 4,
dadurch gekennzeichnet, daß
das Federelement (9) als eine sich parallel zur Symmetrieebene des Kanales erstreckende Stabfeder bzw. Blattfeder ausgebildet ist.

6. Verankerungselement nach Anspruch 5,
dadurch gekennzeichnet, daß
die Feder (9) zur offenen Seite des Kanales hin konvex ausgebildet ist.

7. Verankerungselement nach Anspruch 6,
dadurch gekennzeichnet, daß
die Feder (9) an einem Kanalrand angeschweißt ist.

8. Verankerungselement nach einem der Ansprüche 1 bis 7,
dadurch gekennzeichnet, daß
im Kanalgrund eine der Form der Feder angepaßte Ausnehmung zum Hineindrücken der Feder in diese aufweist.

9. Verankerungselement nach einem der Ansprüche 1 bis 8,
dadurch gekennzeichnet, daß
der Grund der U-förmigen Ausnehmung in seiner Richtung senkrecht zum U-förmigen Querschnitt einen gewölbten Abschnitt aufweist.

10. Verankerungselement nach Anspruch 9,
dadurch gekennzeichnet, daß
die Ränder des Grundes nach außen zum Schaft hin abfallend ausgebildet sind.

11. Verankerungselement nach einem der Ansprüche 1 bis 10,
dadurch gekennzeichnet, daß
koaxial zum ersten Innengewinde im Grund (5) des Kanales eine Ausnehmung vorgesehen ist.

12. Verankerungselement nach einem der Ansprüche 1 bis 11,
dadurch gekennzeichnet, daß
die Wölbung einen zur Mitte hin vertieften Abschnitt aufweist.

13. Verankerungselement nach Anspruch 12,
dadurch gekennzeichnet, daß
die Wölbung jeweils in einem Abstand von der Mitte einen hervorstehend gewölbten Abschnitt aufweist.

14. Verankerungselement nach Anspruch 12,
dadurch gekennzeichnet, daß
der Abstand eines gewölbten Abschnittes (21, 22) in wesentlichen gleich der Mitte (23) zwischen dem Radius des Innengewindes (8) und des Außengewindes (11) ist.

15. Verankerungselement nach einem der Ansprüche 10 bis 14,
dadurch gekennzeichnet, daß
die Feder auf den nach außen hin abfallenden Rändern aufliegt.

16. Verankerungselement nach einem der Ansprüche 1 bis 15,
dadurch gekennzeichnet, daß
der Stab eine strukturierte Oberfläche aufweist.

17. Verankerungselement nach Anspruch 16,
dadurch gekennzeichnet, daß
die Oberfläche ein Gewinde oder ein Kreuzgewinde oder sich in Umfangsrichtung erstreckende Rillen aufweist.

18. Verankerungselement nach Anspruch 16,
dadurch gekennzeichnet, daß
die Oberfläche auf einer Seite einen sich parallel zur Stabachse erstreckenden Zahnabschnitt aufweist.

19. Verankerungselement nach einem der Ansprüche 1 bis 18,
dadurch gekennzeichnet, daß
das Mutternelement und/oder das Fixierelement auf seiner dem Stab zugewandten Seite einen mit der Struktur in Eingriff bringbaren Abschnitt aufweist.

20. Verankerungselement nach Anspruch 19,
dadurch gekennzeichnet, daß
der Abschnitt als hervorstehender Rand ausgebildet ist.

21. Verankerungselement nach einem der Ansprüche 1 bis 20,
dadurch gekennzeichnet, daß
auf dem Kanalgrund wenigsten ein von dessen Fläche zum offenen Ende des Kanalgrundes hin hervorstehender Ansatz vorgesehen ist.

22. Verankerungselement nach Anspruch 21,
dadurch gekennzeichnet, daß
das Fixierelement ein in koaxialer Richtung zu dem Stab federvorgespanntes Element aufweist, welches den Stab zum Kanalgrund hin vorspannt.

23. Verankerungselement nach Anspruch 22,
dadurch gekennzeichnet, daß
das Fixierelement einen Anschlag zur Begrenzung des Einschraubweges in den Kanal hinein aufweist.

24. Verankerungselement nach Anspruch 23,
dadurch gekennzeichnet, daß
zur endgültigen Fixierung ein Schraubelement ohne Anschlag vorgesehen ist.

25. Verankerungselement nach einem der Ansprüche 1 bis 24,
dadurch gekennzeichnet, daß
der Schaft als Gewindeschraube bzw. als Knochenhaken ausgebildet ist.

26. Verankerungselement nach einem der Ansprüche 1 bis 25,
dadurch gekennzeichnet, daß
der den Stab (9) aufnehmende Teil des Kopfes und der Schaft (102) gelenkig miteinander verbunden sind.

27. Verankerungselement nach einem der Ansprüche 15 bis 26,
dadurch gekennzeichnet, daß
die Oberfläche des Stabes (91) eine schraubenförmige Wellung aufweist.

28. Justierwerkzeug für ein Verankerungselement, das einen zur Knochenverankerung bestimmten Schaft (102) und einen mit einem Stab (91) verbindbaren Kopf (103), welcher einen im wesentlichen U-förmigen Querschnitt aufweist und an seinem Grund mit dem Schaft (102) verbunden ist und welcher ferner zwei einen Kanal zur Aufnahme des Stabes bildende und ein Innengewinde (110) aufweisende Schenkel (108, 109) aufweist,
mit
einem Gehäuse mit einem Mantel (151) und einer Stirnseite (152), einem Außengewinde (153) in einem an die Stirnseite (152) angrenzenden Bereich des Mantels (151), welches dem Innengewinde (110) der Schenkel (108, 109) entspricht,
einer Öffnung (154) in der Stirnseite (152) und einer an diese angrenzende Bohrung (155),
einem Druckteil (158) und einer im Inneren der Bohrung (155) angeordneten und auf das Druckteil (158) einwirkenden Druckfeder (161), die das Druckteil (158) so vorspannen kann, daß dieses aus der Öffnung (154) nach außen hervorstehen kann, so daß die Druckfeder (161) mit ihrer Federkraft auf den Stab einwirken kann.

29. Justierwerkzeug nach Anspruch 28,
dadurch gekennzeichnet, daß
das Gehäuse zylinderförmig ausgebildet ist und an seinem der Stirnseite (152) abgewandten Ende einen Abschnitt Ineingriffgelangen mit einem Schraubwerkzeug aufweist.

30. Justierwerkzeug nach Anspruch 28 oder 29,
dadurch gekennzeichnet, daß
die Öffnung (154) in der Stirnseite (152) des Gehäuses kreisförmig ausgebildet ist und daß der Durchmesser der Öffnung (154) kleiner ist als ein Durchmesser der Bohrung (155).

31. Justierwerkzeug nach Anspruch 30,
dadurch gekennzeichnet, daß
das Druckteil (158) einen in der Bohrung (155) angeordneten ersten zylinderförmigen Abschnitt (159), dessen Durchmesser so bemessen ist, daß das Druckteil in der Bohrung (155) in axialer Richtung gleitet und einen daran angrenzenden und sich durch die Öffnung (154) hindurch erstreckenden zweiten zylinderförmigen Abschnitt (160) aufweist, dessen Durchmesser kleiner als der Durchmesser der Öffnung (154) ist und welcher durch die Öffnung (154) nach außen hervorragt, so daß das Druckteil (158) in dem vorgespannten Zustand an einer der Bohrung (155) zugewandten Seite der Stirnseite (152) des Gehäuses anliegt.

32. Justierwerkzeug nach Anspruch 31 oder 32,
dadurch gekennzeichnet, daß
die Oberfläche des aufzunehmenden Stabes (91) ein Gewinde, insbesondere ein Rundgewinde (25) aufweist.

33. Justierwerkzeug nach Anspruch 31 oder 32 ,
dadurch gekennzeichnet, daß
der zweite zylinderförmige Abschnitt (160) des Druckteiles (158) an der Stirnseite seines aus der Öffnung (154) hervorstehenden Endes einen ringförmigen Vorsprung (160a) aufweist.

34. Justierwerkzeug nach Anspruch 33,
dadurch gekennzeichnet, daß
die Abmessungen des ringförmigen Vorsprunges (160a) so gewählt sind, daß der ringförmige Vorsprung (160a) in das Gewinde (25) des Stabes (91) eingreifen kann.

35. Justierwerkzeug nach einem der Ansprüche 28 bis 34,
dadurch gekennzeichnet, daß das Gehäuse an seinem der Stirnseite (152) gegenüber liegenden Ende offen ist und in einem an diesem Ende angrenzenden Bereich des Mantels (151) ein Innengewinde (164) vorgesehen ist.

36. Justierwerkzeug nach Anspruch 35,
dadurch gekennzeichnet, daß
an der dem Druckteil (158) abgewandten Ende der Druckfeder (161) ein in der Bohrung (155) angeordnetes und auf die Druckfeder (161) einwirkendes Druckelement (162) und ein auf das Druckelement (162) einwirkendes Arretierelement in Form einer mit dem Innengewinde (164) des Mantels in Eingriff gelangenden Gewindeschraube (163) vorgesehen sind.

37. Justierwerkzeug nach einem der Ansprüche 28 bis 36,
dadurch gekennzeichnet, daß
die Druckfeder durch übereinander liegende Tellerfedern (161) gebildet ist.

38. Justierwerzeug nach einem der Ansprüche 28 bis 37,
dadurch gekennzeichnet, daß
die Vorspannung der Druckfeder (161) einstellbar ist.

39. Justierwerkzeug zum Justieren der relativen Lage eines Stabes (91) zu dem Aufnahmeteil (104) einer an sich bekannten Polyaxialschraube mit
einem Gehäuse mit einem Mantel (151) und einer Stirnseite (152), einem Außengewinde (153) in einem an die Stirnseite (152) angrenzenden Bereich des Mantels (151), welches dem Innengewinde des Aufnahmeteiles (104) entspricht,
einer Öffnung (154) in der Stirnseite (152) und einer an diese angrenzende Bohrung (155),
einem Druckteil (158) und einer im Inneren der Bohrung (155) angeordneten und auf das Druckteil (158) einwirkenden Druckfeder (161), die das Druckteil (158) so vorspannen kann, daß dieses aus der Öffnung (154) nach außen hervorstehen kann, so daß die Druckfeder (161) mit ihrer Federkraft auf den Stab einwirken kann.

## Claims

1. Anchoring member comprising a shaft (2, 12) to be anchored in a bone and a head (3, 53) for connection with a rod (9, 59), the head having a substantially U-shaped cross-section with a base (5, 55) connected to the shaft (2, 12) and two free legs (6, 7; 56, 57) forming a channel for receiving the rod (9, 59),
whereby the legs have an internal screw thread (8, 58) and an external screw thread (8', 58'),
and a nut member (11, 61) embracing the outer side of the legs (6, 7; 56, 57) and cooperating with the external screw thread and a locking member (10) comprising a thread cooperating with the internal screw thread of the legs,
characterized in that a spring member (19, 79) acting with its elastic force onto the rod (9, 59) to be received is provided.

2. Anchoring member according to claim 1,
characterized in that a spring member (19, 79) is provided at the base of the channel.

3. Anchoring member according to claim 1,
characterized in that the spring member (19, 79) is biased in a direction parallel to the shaft axis.

4. Anchoring member according to claim 3,
characterized in that the spring member (9) is biased towards the open end of the channel.

5. Anchoring member according to any of the claims 1 to 4,
characterized in that the spring member (9) is formed as a rod spring or leaf spring extending parallel to the axis of symmetry of the channel.

6. Anchoring member according to claim 5,
characterized in that the spring (9) is convex towards the open side of the channel.

7. Anchoring member according to claim 6,
characterized in that the spring (9) is welded to an edge of the channel.

8. Anchoring member according to any of the claims 1 to 7,
characterized in that a recess adapted to the shape of the spring is provided in the base of the channel for pressing the spring into the recess.

9. Anchoring member according to any of the claims 1 to 8,
characterized in that the base of the U-shaped recess has a cambered portion in its direction perpendicular to the U-shaped cross-section.

10. Anchoring member according to claim 9,
characterized in that the edges of the base fall away outwardly towards the shaft.

11. Anchoring member according to any of the claims 1 to 10,
characterized in that a recess is provided in the base (5) of the channel, the recess being coaxial with the first internal screw thread.

12. Anchoring member according to any of the claims 1 to 11,
characterized in that the camber comprises a portion which is depressed towards the center.

13. Anchoring member according to claim 12,
characterized in that the camber comprises respective projecting cambered portions spaced from the center.

14. Anchoring member according to claim 12,
characterized in that the distance of a cambered portion (21, 22) corresponds substantially to the center (23) between the radius of the internal screw thread (8) and the external screw thread (11).

15. Anchoring member according to any of the claims 10 to 14,
characterized in that the spring rests on the outwardly sloping edges.

16. Anchoring member according to any of the claims 1 to 15,
characterized in that the rod has a textured surface.

17. Anchoring member according to claim 16,
characterized in that the surface comprises a thread or a cross-thread or grooves extending in peripheral direction.

18. Anchoring member according to claim 16,
characterized in that the surface comprises a rack on one side thereof, the rack extending parallel to the rod axis.

19. Anchoring member according to any of the claims 1 to 18,
characterized in that the nut member and/or the locking member comprises a portion on its side facing the rod for engaging the texture.

20. Anchoring member according to claim 19,
characterized in that the portion is formed as a projecting edge.

21. Anchoring member according to any of the claims 1 to 20,
characterized in that the channel base has at least one nose projecting from the base surface towards the open end of the channel base.

22. Anchoring member according to claim 21,
characterized in that the locking member comprises a member which is spring-biased in coaxial direction towards the rod and preloads the rod towards the channel base.

23. Anchoring member according to claim 22,
characterized in that the locking member comprises a stop for limiting the screwing path into the channel.

24. Anchoring member according to claim 23,
characterized in that a screw member without stop is provided for the final lock.

25. Anchoring member according to any of the claims 1 to 24,
characterized in that the shaft is formed as threaded screw or as bone hook, resp.

26. Anchoring member according to any of the claims 1 to 25,
characterized in that the portion of the head receiving the rod (9) is hingedly connected to the shaft (102).

27. Anchoring member according to any of the claims 15 to 26,
characterized in that the surface of the rod (91) comprises a wavy screw thread.

28. Adjustment tool for an anchoring member which comprises a shaft (102) for anchoring in a bone and a head (103) for connection with a rod (91), the head comprising a substantially U-shaped cross-section and being connected, at its base, with the shaft (102), the head further comprising two legs (108, 109) forming a channel for receiving the rod and having an internal screw thread (110),
the adjustment tool comprising a housing having a jacket (151) and a face (152), an external screw thread (153) formed at a region of the jacket (151) adjacent to the face (152) and corresponding to the internal screw thread (110) of the legs (108, 109),
an aperture (154) in the face (152) and a bore (155) adjacent thereto,
a pressure piece (158) and a compression spring (161) which is disposed within the bore (155) and acts onto the pressure piece (158), the compression spring being adapted to preload the pressure piece (158) so as to be able to project outwardly from the aperture (154), whereby the compression spring (161) can, with its elastic force, act onto the rod.

29. Adjustment tool according to claim 28,
characterized in that the housing is cylindrical and has, at its end opposite to the face (152), a portion for engagement with a screwing tool.

30. Adjustment tool according to claims 28 or 29,
characterized in that the aperture (154) in the face (152) of the housing is circular and the diameter of the aperture (154) is smaller than a diameter of the bore (155).

31. Adjustment tool according to claim 30,
characterized in that the pressure piece (158) comprises a first cylindrical portion (159) which is located within the bore (155) and has a diameter sized to allow the pressure piece to slide in axial direction within the bore (155), and a following second cylindrical portion (106) which extends through the aperture (154) and has a diameter which is smaller than the diameter of the aperture (154) so that the second cylindrical portion projects outwardly through the aperture (154), whereby in the preloaded state the pressure piece (158) abuts the side of the face (152) of the housing facing the bore (155).

32. Adjustment tool according to claim 31 or 32,
characterized in that the surface of the rod (91) to be received comprises a thread, in particular a round thread (25).

33. Adjustment tool according to claim 31 or 32,
characterized in that the second cylindrical portion (160) of the pressure piece (158) has an annular projection (160a) formed at the face of its end projecting from the aperture (154).

34. Adjustment tool according to claim 33,
characterized in that the dimensions of the annular projection (160a) are so that the annular projection (160a) may engage the thread (25) of the rod (91).

35. Adjustment tool according to any of the claims 28 to 34,
characterized in that the housing is open at its end opposite to the face (152) and an internal screw thread (164) is provided in a region of the jacket (151) adjacent to this end.

36. Adjustment tool according to claim 35,
characterized in that a pressure member (162) disposed within the bore (155) and acting upon the compression spring (161) is provided at the end of the compression spring (161) opposite to the pressure piece (158) and a locking member acting upon the pressure member (162) is provided in the form of a threaded screw (163) engaging the internal screw thread (164) of the jacket.

37. Adjustment tool according to any of the claims 28 to 36,
characterized in that the compression spring is formed by stacked disk springs (161).

38. Adjustment tool according to any of the claims 28 to 37,
characterized in that the preload of the compression spring (161) is adjustable.

39. Adjustment tool for adjusting the position of a rod (91) relative to the seat part (104) of a per se known polyaxial screw,
comprising a housing having a jacket (151) and a face (152), an external screw thread (153) formed at a region of the jacket (151) adjacent to the face (152) and corresponding to the internal screw thread of the seat part (104),
an aperture (154) in the face (152) and a bore (155) adjacent thereto,
a pressure piece (158) and a compression spring (161) disposed in the interior of the bore (155) and being adapted to act upon the pressure piece (158) to preload the pressure piece (158) so as to be able to project outwardly from the aperture (154), whereby the compression spring (161) can, with its elastic force, act onto the rod.

## Revendications

1. Élément d'ancrage comprenant un corps (2, 12) destiné à l'ancrage des os et une tête (3, 53) qui peut être assemblée avec une tige (9, 59) et présente une section sensiblement en forme de U, dont le fond (5, 55) est assemblé avec le corps (2, 12) et qui est munie de deux branches libres (6, 7 ; 56, 57) formant un canal destiné à recevoir la tige (9, 59), les branches étant munies d'un taraudage (8, 58) et d'un filet extérieur (8', 58'), et comprenant un élément à écrou (11, 61) entourant par l'extérieur les branches (6, 7 ; 56, 57) et muni d'un taraudage coopérant avec le filet extérieur, et un élément de blocage (10) muni d'un filet coopérant avec le taraudage des branches, caractérisé en ce qu'il est prévu un élément à ressort (19, 79) agissant par son effet de ressort sur la lige (9, 59) à recevoir.

2. Élément d'ancrage selon la revendication 1, caractérisé en ce qu'il est prévu un élément à ressort (19, 79) sur le fond du canal.

3. Élément d'ancrage selon la revendication 1, caractérisé en ce que l'élément à ressort (19, 79) est précontraint dans un sens parallèle à l'axe du corps de vis.

4. Élément d'ancrage selon la revendication 3, caractérisé en ce que l'élément à ressort (9) est précontraint en direction de l'extrémité ouverte du canal.

5. Élément d'ancrage selon l'une quelconque des revendications 1 à 4, caractérisé en ce que l'élément à ressort (9) est conçu en forme de ressort en tige ou ressort à lames qui s'étend parallèlement au plan de symétrie du canal.

6. Élément d'ancrage selon la revendication 5, caractérisé en ce que le ressort (9) est conçu avec une courbure orientée vers l'extrémité ouverte du canal.

7. Élément d'ancrage selon la revendication 6, caractérisé en ce que le ressort (9) est soudé contre un bord du canal.

8. Élément d'ancrage selon l'une quelconque des revendications 1 à 7, caractérisé en ce que le fond du canal est muni d'un évidement adapté à la forme du ressort et dans lequel est poussé le ressort.

9. Élément d'ancrage selon l'une quelconque des revendications 1 à 8, caractérisé en ce que le fond de l'évidement en forme de U, dans sa direction perpendiculaire à la section en forme de U, comporte une partie cintrée.

10. Élément d'ancrage selon la revendication 9, caractérisé en ce que les bords du fond sont conçus en retombant vers l'extérieur en direction du corps de vis.

11. Élément d'ancrage selon l'une quelconque des revendications 1 à 10, caractérisé en ce qu'il est prévu un évidement réalisé dans le fond (5) du canal dans le sens coaxial par rapport au premier taraudage.

12. Élément d'ancrage selon l'une quelconque des revendications 1 à 11, caractérisé en ce que la courbure comporte une partie plus profonde vers le milieu.

13. Élément d'ancrage selon la revendication 12, caractérisé en ce que la courbure comporte de part et d'autre, à une distance donnée du milieu, une partie cintrée en saillie.

14. Élément d'ancrage selon la revendication 12, caractérisé en ce que la distance d'une partie cintrée (21, 22) est sensiblement égale au centre (23) entre le rayon du taraudage (8) et le rayon du filet extérieur (11).

15. Élément d'ancrage selon l'une quelconque des revendications 10 à 14, caractérisé en ce que le ressort est en appui sur les bords retombant vers l'extérieur.

16. Élément d'ancrage selon l'une quelconque des revendications 1 à 15, caractérisé en ce que la tige présente une surface structurée.

17. Élément d'ancrage selon la revendication 16, caractérisé en ce que la surface est munie d'un filet ou d'un filet à hélice croisée ou de rainures qui s'étendent dans le sens périphérique.

18. Élément d'ancrage selon la revendication 16, caractérisé en ce que la surface est formée sur un côté d'une partie dentée qui s'étend parallèlement à l'axe de la tige.

19. Élément d'ancrage selon l'une quelconque des revendications 1 à 18, caractérisé en ce que l'élément à écrou et/ou l'élément de blocage est muni, sur son côté orienté vers la tige, d'une partie pouvant entrer en prise avec la structure.

20. Élément d'ancrage selon la revendication 19, caractérisé en ce que la partie est conçue comme un bord en saillie.

21. Élément d'ancrage selon l'une quelconque des revendications 1 à 20, caractérisé en ce qu'il est prévu sur le fond du canal au moins une saillie qui s'avance au-delà de la surface de celui-ci vers l'extrémité ouverte du fond du canal.

22. Élément d'ancrage selon la revendication 21, caractérisé en ce que l'élément de blocage est muni d'un élément précontraint dans le sens coaxial par rapport à la tige, lequel exerce une précontrainte sur la tige en direction du fond du canal.

23. Élément d'ancrage selon la revendication 22, caractérisé en ce que l'élément de blocage est muni d'une butée destinée à limiter sa profondeur de vissage dans le canal.

24. Élément d'ancrage selon la revendication 23, caractérisé en ce que pour le blocage final il est prévu un élément de vis sans butée.

25. Élément d'ancrage selon l'une quelconque des revendications 1 à 24, caractérisé en ce que le corps de vis est conçu comme un boulon ou un crochet pour os.

26. Élément d'ancrage selon l'une quelconque des revendications 1 à 25, caractérisé en ce que la partie de la tête de vis recevant la tige (9) et le corps de vis (102) sont assemblés de manière articulée l'un par rapport à l'autre.

27. Élément d'ancrage selon l'une quelconque des revendications 15 à 26, caractérisé en ce que la surface de la tige (91) est une surface ondulée en forme d'hélice.

28. Dispositif d'ajustage destiné à un élément d'ancrage comprenant un corps (102) destiné à l'ancrage des os et une tête (103) qui peut être assemblée avec une tige (91) et présente une section sensiblement en forme de U, dont le fond est assemblé avec le corps (102) et qui, en outre, est munie de deux branches (108, 109) formant un canal destiné à recevoir la tige et muni d'un taraudage (108, 109), lequel dispositif comprend un boîtier avec une paroi cylindrique (151) et une face frontale (152), un filet extérieur (153) réalisé dans une zone de la paroi (151) contiguë à la face frontale (152), lequel correspond au taraudage (110) des branches (108, 109), un orifice (154) réalisé dans la face frontale (152) et une forure (155) juxtaposé audit orifice, un organe de pression (158) et un ressort de pression (161) disposé à l'intérieur de la forure (155) et agissant sur l'organe de pression (158), lequel ressort de pression permet d'exercer une précontrainte sur l'organe de pression (158) de telle sorte que celui-ci s'avance vers l'extérieur hors de l'orifice (154), de telle sorte que le ressort de pression (161) puisse agir par son effet de ressort sur la tige.

29. Dispositif d'ajustage selon la revendication 28, caractérisé en ce que le boîter est un boîtier cylindrique et comporte sur son extrémité opposée à la face frontale (152) une partie destinée à entrer en prise avec un outil de vissage.

30. Dispositif d'ajustage selon la revendication 28 ou 29, caractérisé en ce que l'orifice (154) est un orifice circulaire dans la face frontale (152) du boîtier et en ce que le diamètre de l'orifice (154) est inférieur au diamètre de la forure (155).

31. Dispositif d'ajustage selon la revendication 30, caractérisé en ce que l'organe de pression (158) comporte une première partie cylindrique (159), dont le diamètre est choisi de telle sorte que l'organe de pression puisse se décaler dans le sens axial dans la forure (155), et une deuxième partie cylindrique (160) dans le prolongement de celle-ci, qui s'étend à travers l'orifice (154) et dont le diamètre est légèrement inférieur au diamètre de l'orifice (154) et lequel s'avance en saillie vers l'extérieur à travers l'orifice (154), de telle sorte que l'organe de pression (158), à l'état précontraint, est en appui contre un côté, orienté vers la forure (155), de la face frontale (152) du boîtier.

32. Dispositif d'ajustage selon la revendication 31 ou 32, caractérisé en ce que la surface de la tige (91) à recevoir est munie d'un filet, en particulier un filet arrondi (25).

33. Dispositif d'ajustage selon la revendication 31 ou 32, caractérisé en ce que la deuxième partie cylindrique (160) de l'organe de pression (158) est munie d'une saillie annulaire (160a) réalisée sur la face frontale de son extrémité en saillie hors de l'orifice (154).

34. Dispositif d'ajustage selon la revendication 33, caractérisé en ce que les dimensions de la saillie annulaire (160a) sont choisies de telle sorte que la saillie annulaire (160a) puisse entrer en prise avec le filet (25) de la tige (91).

35. Dispositif d'ajustage selon l'une quelconque des revendications 28 à 34, caractérisé en ce que le boîtier est ouvert sur son extrémité située en face de la face frontale (152) et un taraudage (164) est prévu dans une zone de la paroi (151), contiguë à cette extrémité.

36. Dispositif d'ajustage selon la revendication 35, caractérisé en ce que, sur l'extrémité du ressort de pression (161) opposée à l'organe de pression (158), il est prévu un élément de pression (162) disposé dans la forure (155) et agissant sur le ressort de pression (161), et un élément d'arrêt agissant sur l'élément de pression (162) en forme de boulon fileté (163) entrant en prise avec le taraudage (164) de la paroi.

37. Dispositif d'ajustage selon l'une quelconque des revendications 28 à 36, caractérisé en ce que le ressort de pression est formé par des ressorts à lames (161) disposés les uns au-dessus des autres.

38. Dispositif d'ajustage selon l'une quelconque des revendications 28 à 37, caractérisé en ce que la précontrainte du ressort de pression (161) est réglable.

39. Dispositif d'ajustage destiné à ajuster la position d'une tige (91) par rapport à l'organe de réception (104) d'une vis polyaxiale connue en soi, comprenant un boîtier avec une paroi (151) et une face frontale (152), un filet extérieur (153) réalisé dans une zone de la paroi (151) contiguë à la face frontale (152), lequel correspond au taraudage (110) de l'organe de réception (104), un orifice (154) réalisé dans la face frontale (152) et une forure (155) juxtaposée audit orifice, un organe de pression (158) et un ressort de pression (161) disposé à l'intérieur de la forure (155) et agissant sur l'organe de pression (158), lequel ressort de pression permet d'exercer une précontrainte sur l'organe de pression (158) de telle sorte que celui-ci s'avance vers l'extérieur hors de l'orifice (154), de telle sorte que le ressort de pression (161) puisse agir par son effet de ressort sur la tige.
